# EUROPÄISCHE PATENTSCHRIFT

(11) **EP 1 288 182 B1**
(45) Veröffentlichungstag und Bekanntmachung des Hinweises auf die Patenterteilung: **01.12.2010**
(21) Anmeldenummer: 00966606.6
(22) Anmeldetag: 21.09.2000
(51) Int. Cl.: C07C 2/76, C07C 11/04, C07C 11/24, C10G 15/08, C10G 15/12

(54) **VERFAHREN ZUR DIREKTEN METHANPYROLYSE**
METHOD FOR DIRECT METHANE PYROLYSIS
PROCEDE DE PYROLYSE DIRECTE DU METHANE

(30) Priorität: 21.03.2000 RU 2000106774
(43) Veröffentlichungstag der Anmeldung: 05.03.2003
(73) Patentinhaber: JSC "National gaz company", Moscow 117105 (RU)
(72) Erfinder: GENKIN, Vladimir Naumovich, Nizhny Novgorod, 603039 (RU); GENKIN, Mikhail Vladimirovich, Nizhny Novgorod, 603000 (RU); TYNNIKOV, Jury Georgievich, Nizhny Novgorod, 603163 (RU)
(74) Vertreter: Jeck, Anton
(86) Internationale Anmeldenummer: PCT/RU2000/000375
(87) Internationale Veröffentlichungsnummer: WO 2001/070656

(56) Entgegenhaltungen:
- DD-A- 157 414
- DE-A1- 2 718 076
- DE-B- 1 012 899
- FR-A- 2 596 046
- FR-A1- 2 320 977
- SU-A- 956 545
- US-A- 3 156 734

## Beschreibung

### Erfindungsgebiet

Die vorliegende Erfindung bezieht sich auf die Gewinnung von Kohlenwasserstoffen mit geringerer Anzahl von Kohlenstoffatomen in den Molekülen und ist für die Gewinnung von Äthylen, Azetylen und anderen unteren Olefinen aus Methan bestimmt.

### Stand der Technik

Die Massennachfrage nach der Erzeugung der unteren Olefine, insbesondere nach Äthylen (jährliche Nachfrage etwa 10³t), als Grundlage der organischen Synthese der chemischen Industrie bestimmt verschiedene Verfahren zur Gewinnung dieser Olefine.

Das Hauptverfahren der Äthylengewinnung ist heutzutage die Hochtemperaturspaltung (Pyrolyse) der Kohlenwasserstoffe, wie die flüssige Öldestillaten, oder der unteren Paraffinkohlenwasserstoffe.

Hier ist das vereinfachte Reaktionsschema:

CₙH₂ₙ₊₂ ↔ CₙH₂ₙ + H₂ₒ

Die Verwendung teurer Ausgangsmaterialien zieht aber auch hohe Produktionskosten nach sich.

Es gibt mehrere bekannte Verfahren der Gewinnung von unteren Olefinen aus einem billigerem Produkt, nämlich Methan, durch chemische Konversion von methanhaltigern (gegen 98 %) Erdgas.

So ist ein Verfahren der Äthylengewinnung bekannt, das bei vorhandenem Stickstoffsuboxid (0,56 - 4,0) ausgeführt wird: bei Temperatur von 200 - 415°C des Katalysators wird HZSM-5 Zeolith mit 35 - 55 Silikatmodul genommen, oder es wird dasselbe Zeolith, modifiziert mit 0,1 - 5 Mas. % des Übergangsmetalls Fe bzw. Co oder eines Seltenenerdenmetalls bzw. deren Gemisch, oder dasselbe Zeolith, modifiziert mit 2- 15 % Gemisch aus Zink- und Chromoxid (im Verhältnis 1:1-3), oder dasselbe Zeolith, modifiziert mit 3 - 5 % Phosphorfünfoxid genommen. Bei diesem Verfahren wird ein Durchflussreaktor verwendet, dem das oben genannte Gemisch unter einem Druck von 2,2 10⁵ Pa und einer Geschwindigkeit von 0,4 10⁻⁵ m³/s zugeführt wird. [Beschreibung zum Urheberschein Nº 1353768, veröff. Nº 43 23.11.97].

Aus der Beschreibung ist ersichtlich, dass die Aufgabenlösung auf die Ausarbeitung verschiedener Varianten teurer Katalysatoren gerichtet ist, weil bei den angegebenen Reaktionsbedingungen (Druck, Temperatur, Geschwindigkeit der Gasgemischzufuhr) ohne katalytische Initiierung eine Konversion von Methan in andere Kohlenwasserstoffe, insbesondere in Äthylen, wegen seiner Beständigkeit nicht erfolgen kann.

Außerdem macht die Verwendung der genannten Katalysatoren die Behandlung des methanhaltigen Gases komplizierter und teurer und vernichtet alle Vorteile der Verwendung eines billigeren Ausgangsmaterials.

Nach dem bekannten Verfahren der Thermooxidationsmethanpyrolyse wird in die Mischkammer mit Überschallgeschwindigkeit Methan (unter einem Druck von 300 Atm. Und auf 700 - 900 °C aufgeheizt) und Sauerstoff (unter niedrigerem Druck und auf 150 - 200 °C aufgeheizt) zugeführt. Nach der Vermischung der Komponenten wird das gewonnene Methan-Sauerstoff-Gemisch mit Überschallgeschwindigkeit in eine weitere Kammer zum gasdynamischen Bremsen des Gemisches gerichtet. Infolge des Bremsens zündet das Gemisch bei diesem Druck und einer Temperatur von 1400- 1500 °C selbst, und es verbrennt in der nächsten Brennkammer. Die Reaktionsprodukte werden dann abgekühlt ("gehärtet") und entnommen. [Beschreibung zum Urheberschein Nº 1778146, veröff. Nº 44, 30.11.1992].

Aus der Beschreibung ist ersichtlich, dass bei der Thermooxidationsmethanpyrolyse bei einem Sauerstoffverbrauchsfaktor von 0,28 die Brenntemperatur nach der Selbstentzündung im Diffusor etwa 1400-1500 °C beträgt.

Das nach der Wasserhärtung gewonnene Pyrogas enthält etwa 10 Vol.% Azetylen unter einem Druck von 10 - 13 Atm., das Restliche ist Kohlenwasserstoffoxid und Wasserstoff.

Demgemäß stellen die angeführten Bedingungen der Thermooxidationsmethanpyrolyse nur eine geringe Ausbeute von Azetylen sicher.

Die Äthylengewinnung aus Azetylen erschwert den Vorgang und erfordert zusätzliche Ausgaben.

Im Bezug auf die vorliegende Erfindung hinsichtlich der gestellten Aufgabe, nämlich die Verwendung eines billigen Ausgangsmaterials, methanhaltiges Gases, ist das Verfahren der direkten Methankonversion, das in dem plasmachemischen Reaktor durch Aufheizen von plasmabildenden Gasen, wie Erdgas oder Wasserstoff ausgeführt wird, die beste Lösung. Der Wasserstoff wird in einem Elektrolichtbogen auf 3500 - 4000 aufgeheizt, dann wird in die Reaktionszone das auf 300 - 900 K vorgeheizte, zu pyrolysierende Gas zugeführt. Der Pyrolysevorgang verläuft bei einer Temperatur von 2200 - 2400 K unter einem Druck von 10⁴ Pa-10⁵ Pa in 2,5 10⁻³ -3 10⁻³s.

Im beschriebenen Verfahren wird angenommen, dass die Reaktionen bei thermischer Methanzersetzung nach dem Kasselschema verlaufen:

CH₄ → C₂H₆ →C₂H₄→C₂H₂

Dabei sind Azetylen und eine geringe Äthylenmenge die gewonnenen Hauptprodukte.

Es ist festgestellt worden, dass das beste Methan-Wasserstoff-Verhältnis bei der Erdgasverarbeitung im Wasserstoffplasma CH₄/H₂ = 2 ist.

Der größte Azetylenvolumenanteil in den Pyrolyseprodukten erreicht in diesem Fall 15,55 Vol.% (der Methan-Azetylen-Konversionsgrad beträgt dabei 73 %, und der Gesamtmethankonversionsgrad ist 80 %. Unter diesen Bedingungen ist der Energieverbrauch 32,4 - 37,4 mJ pro 1 m³. Wenn die Vorgangsbedingungen optimal sind, dann ist der Durchschnittsgehalt der Methanpyrolyseprodukte wie folgt (Vol.%): Wasserstoff - 75,9; Azetylen- 15,55; Äthylen - 0,527; Propylen- 0,0153; Diazetylen - 0,39; Vinylazetylen - 0,022; H-Butan - Spuren; Kohlenwasserstoffoxid - Spuren; Stickstoff - 0,5.

Danach wird eine kombinierte Pyrolysenprodukthärtung durchgeführt. (Vorgänge und Geräte der plasmachemischen Technologie. Kiew. Hochschule. 1979. Seite 221-227)

Es ist aus den angeführten Ergebnissen ersichtlich, dass die direkte Methanzersetzung bei den obengenannten Bedingungen hauptsächlich zur Bildung von Azetylen und einer geringen Äthylenmenge führt.

Aus der FR 2 596 046 A2 ist ein Verfahren zur Senkung der Temperatur von Plasma bekannt, bei dessen Wechselwirkung mit Methan eine Pyrolyse des Methans erfolgt. Dieser Prozess geschieht in nur einer Etappe, in der eine Erwärmung des Methanplasmas erfolgt, wonach eine Abkühlung folgt.

Schließlich ist aus der DD 157 414 ein Verfahren zur Kohlenwasserstoffpyrolyse mit der Gewinnung von Azetylen bzw. eine Pyrolyse eines Kohlenwasserstoffgernisches mit Stickstoff und der Erzeugung von Blausäure bekannt. Aus dieser Schrift ist auch ein Verfahren zur Durchführung von endothermischen Reaktionen (Pyrolyse) im Wasserstoffstrahl bekannt, der im Bogen hocherhitzt wird. In dieser Schrift werden keine Zeitintetrvalle für den Prozess und keine Druckbereiche genannt.

Der Erfindung ist die technische Aufgabe zugrunde gelegt, ein Verfahren der direkten Methankonversion zu entwickeln, mit dem sich der Äthylengehalt in den Reaktionsprodukten erhöhen lässt.

### Zusammenfassung der Erfindung

Die gestellte technische Aufgabe wird dadurch gelöst, dass das zur Pyrolyse bestimmte, methanhaltige Gas auf 900 - 1000 K aufgeheizt und die Methankonversion in zwei Stufen durchgeführt wird. Zuerst wird das Gas auf 1800- 2100 K in 1-2 ms aufgeheizt, dann wird das aufgeheizte Primärpyrolyseproduktgemisch innerhalb 5 -10 ms unter Druck 2 - 5 Mpa unter adiabatischer Bedingung gehalten. Danach wird eine Reaktionsprodukthärtung durchgeführt. Dabei wird das obengenannte Temperaturintervall auf der ersten Stufe der Methankonversion durch Außenenergiezufuhr aufrechterhalten. Das Aufheizen des Pyrolysegemisches wird in der ersten Stufe entweder durch Methanaufheizung im Wasserstoffplasmastrahl oder durch Elektrostromaufheizung oder durch ein Induktionsaufheizungsverfahren ausgeführt.

Wesentliche neue Merkmale der beanspruchten Erfindung sind: eine zweistufige Methankonversion, das oben genannte Temperaturintervall der Vorheizung des Pyrolyseausgangsmaterials, die genannten Angaben über Temperatur und Dauer der Gasaufheizung in der ersten Stufe der Methanpyrolyse, sowie die angegebenen Intervalle von Druck- und Haltezeitwerten für die Produkte der Primärpyrolyse unter adiabatischer Bedingung in der zweiten Stufe der Methankonversion.

Die vorläufige Aufheizung des Pyrolyseausgangsmaterials auf 900 - 1000 K stellt seine schnellere Aufheizung in der Grundstufe sicher und trägt dadurch zur tieferen Methankonversion bei.

Die Zweckmäßigkeit der angegebenen Pyrolysebedingungen geht aus folgenden Gründen hervor:
1. Bei einer Temperatur der Methanvorheizung auf der ersten Stufe der Konversion unter 900 K verlängert sich die Zeit der Aufheizung des Methans auf die erforderlichen Temperaturen, was eine Verkohlung des Reaktors nach sich zieht. Die Vorheizung des Methans über 1000 K ist unzweckmäßig, denn die Aufheizung auf 1000 K wird von der Temperatur der Pyrolysenabfallprodukte sichergestellt, und für größere Temperaturen als 1000 K ist eine zusätzliche, komplizierte Ausrüstung nötig.
2. In der ersten Stufe der Methankonversion zieht die Aufheizung auf Temperaturen unter 1800 K in der angegebenen Zeit eine Verringerung der Konversionstiefe und folglich der Ausgangsproduktmenge nach sich. Die Aufheizung auf Temperaturen über 2100 K unter gleichen Bedingungen führt zur Verschiebung der Pyrolysereaktionen in Richtung der Azetylen- und der Rußbildung.
3. Eine Aufheizung auf die erforderlichen Temperaturen in der ersten Methankonversionsstufe innerhalb von 1 ms führt zu einer ungleichmäßigen Gasdurchwärmung, wodurch die Konversionstiefe verringert wird. Die Aufheizung auf die gleichen Temperaturen in der Zeit von über 2 ms verschiebt die Reaktionen in Richtung Azetylenbildung.
4. In der zweiten Konversionsstufe beim Halten des aufgeheizten Gemisches der Primärpyrolyseprodukte unter einem Druck unter 2 Mpa innerhalb der angegebenen Zeit wird eine Ausbeuteerhöhung in den Azetylenreaktionsprodukten beobachtet. Das Halten der gleichen Produkte unter einem Druck von über 5 Mpa innerhalb der gleichen Zeit bedingt eine Verfestigung der Reaktorkonstruktion und eine Erhöhung des Materialeinsatzes.
5. Beim Halten des aufgeheizten Gemisches von Prämierkonversionsprodukten unter dem angegebenen Druck innerhalb der Zeit von weniger als 5 ms steigt in der zweiten Stufe die Azetylenausbeute, weil eine Azetylen-Äthylen-Hydrierungsreaktion nicht zustande kommen kann. Das Halten derselben Produkte unter demselben Druck innerhalb einer Zeit von länger als 10 ms trägt zur Rußbildung und Reaktorverkohlung bei.

### Ausführliche Beschreibung der Erfindung

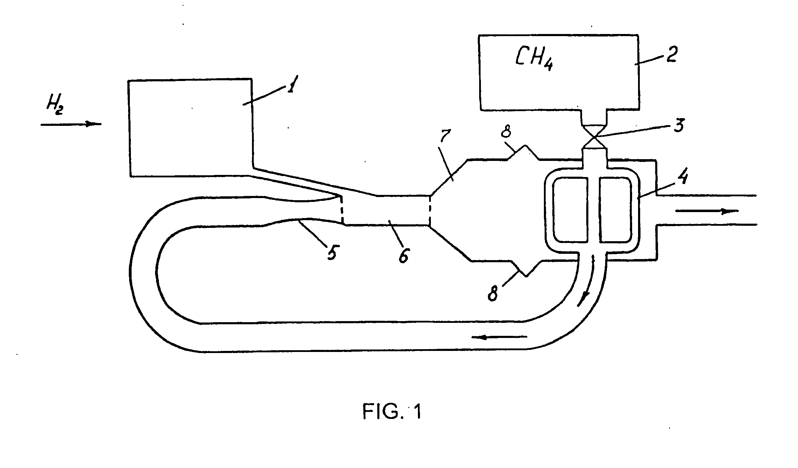

Die Erfindung wird anhand der Zeichnung näher erläutert, auf der das Ausführungsmusterschema der Anlage für die Realisierung der vorliegenden Erfindung dargestellt ist, wobei Methan in einem plasmachemischen Reaktor aufgeheizt wird.

Das oben genannte Verfahren der Pyrolyseausgangsmaterialaufheizung ist deshalb vorteilhaft, weil dadurch die vollständigste und gleichmäßigste Gasaufheizung in kurzer Zeit (1-2 ms) sichergestellt wird.

Die folgenden Bezugszeichen bedeuten:
1 - Niedertemperaturplasmagenerator (Plasmatron);
2 - Methantank;
3 - Gasverbrauchsregler;
4 - Vorheizungskammer;
5 - Lavaldüse;
6 - Zone der ersten Methankonversionsstufe;
7 - Diffusor;
8 - Rußauffänger;

Die Quelle des zu verarbeitenden Ausgangsmaterials (Tank oder Rohrleitung 2) ist durch den Gasverbrauchsregler 3 mit der Vorheizungskammer 4 verbunden, die durch Lavaldüse 5 mit dem Reaktor verknüpft ist. Mit dem Reaktor ist auch ein Plasmatron 1 verbunden. Mit der Zone der ersten Methankonversionsstufe 6 ist ein Diffusor 7 verknüpft, der mit dem Rußauffänger verbunden ist.

Das Methan aus dem Tank 2 (in dem es sich unter einem hohem Druck von 5 -10 MPa befindet) gelangt in die Vorheizungskammer 4, in der das Gas mittels der Abfallgaswärme 11 auf 900 - 1000 K aufgeheizt wird. Danach wird das geheizte Gas durch die Lavaldüse in den Reaktor 6 zur Primärmethankonversion zugeführt. Die Konversion erfolgt durch eine Gasaufheizung auf 1800- 2100 K innerhalb von 1 - 2 ms in Wasserstoffplasma, aufgeheizt im Plasmotron 1 auf 3500 - 4000 K. Für eine effiziente Vermischung von Rohstoff mit Plasma wird ein Wasserstoffsstrahldruck nahe beim Gasstromdruck im Reaktor aufrechterhalten. Mittels der Gasführung durch die Lavaldüse wird der Gasdruck geringer. Das aufgeheizte Gemisch der Primärpyrolyseprodukte wird in den Diffusor gelenkt, in dem ein Bremsen des Gemisches, begleitet von einer starken Drucksteigerung (Druckwiederaufbau auf 2 - 5 mPa) und einem 5 - 10 ms langen Halten im adiabatischem Zustand, erfolgt. Auf dieser Stufe erfolgt die Umwandlung von Azetylen in Äthylen und auch die zusätzliche Äthylenbildung durch allmähliche Stromabkühlung.

Das Gemisch der Pyrolyseprodukte wird der Härtung zugeführt. Zur Verhinderung des Eintritts von Ruß in die Pyrolyseprodukte ist der Rußauffänger 8 vorgesehen. Die Anlage erhält die quasistationären, gasdynamischen, thermischen Arbeitsbedingungen mit einer Zyklusdauer von 10²÷10⁵s.

Die Dauer der transitiven, gasdynamischen und thermischen Bedingungen nach dem Gaseinlassungssignal bis zum Eintreten der Bedingungen mit einem konstanten Verbrauch und einer konstanten Temperatur beträgt nicht mehr als 5 % der Dauer der quasistationären Bedingung.

Die Messanlagen für Temperatur, Druck und Gasgehalt sind auf der Zeichnung nicht angegeben, wurden jedoch verwendet.

Die Ausführung der Anlage ermöglicht eine Demontage und eine Entrußung. In der ersten Konversionsstufe erfolgt in 1 - 2 ms bei Temperaturen von etwa 1800 - 2100 K eine partielle Konversion von Methan in Azetylen (etwa 30 Vol.%) nach folgendem Schema:

CH₄ → C₂H₆ → C₂H₄ → C₂H₂.

Eine Konversionsgradsteigerung ist in dieser Stufe wegen der großen Azetylenbildungsenergie (≈ 400 KJ/Mol) unzweckmäßig.

In der zweiten Stufe verlaufen wegen des hohen Drucks im angegebenen Wertbereich effiziente Reaktionen, die zu Azetylen-Äthylen-Hydrierung nach folgendem Äquivalenzschema führen:

C₂H₂ + H₂ → C₂H₄.

Im Laufe dieser Reaktionen wird teilweise Energie verbraucht, die im während der ersten Stufe gebildeten Azetylen gespeichert wurde; deshalb erfolgt der für den adiabatischen Prozess typische Temperaturabfall etwas langsamer (in 5-10 ms), was die weitere Methankonversion bis zur Äthylenbildung sicherstellt.

Chemische Reaktionen verlaufen also hauptsächlich in der zweiten Stufe der Methankonversion, in der der Strom auf eine minimale Geschwindigkeit abgebremst wird. Das ist wichtig, weil die benötigte Kanallänge sich verringert, die von der Anwesenheitsdauer des Reagens im Strom bestimmt wird. Durch eine Drucksteigerung wird eine Vorgangsselektivität sichergestellt (der Azetylenanteil wird geringer). Außerdem geht der Temperaturabfall, der wegen der Wärmeabsorption bei den chemischen Reaktionen erfolgt, gar nicht so schnell vor sich, weil die Temperatur durch das Stromabbremsen aufrechterhalten wird.

In den Tabellen 1 - 5 sind Angaben über Testwerte des Verfahrens angeführt. Es bedeutet:
T_{H}- Temperatur der Vorheizung des Methan-Wasserstoff-Gemisches, K;
Tₙ - Temperatur der Methanpyrolyse auf der ersten Stufe, K;
τ_{H} - Dauer der Aufheizung des Pyrolyseausgangsmaterials in der ersten Stufe, C;
P - Druck in der ersten Stufe der Methanpyrolyse, MPa;
τ_{B}- Haltedauer des Gemisches der Primärmethanpyrolyseprodukte in der zweiten Stufe.

In allen Fällen betrug der Methanverbrauch 1,2·10⁻³ kg/s.

**Tabelle 1**

| Tₙ = 1950 K; τ_{H} = 1,5 MS; P = 3,5 MPa; τ_{B} =7 MS | | | | | |
|---|---|---|---|---|---|
| Temperatur der Gemischvorheizung, K | Methankonversionsgrad, % | Gehalt der Pyrolyseprodukte, % Mass. | | | |
| | | Wasserstoff | Azetylen | Ethylen | Sonstige Produkte |
| 800 | 79,9 | 10,2 | 12,1 | 57,6 | 20,1 |
| 900 | 81,9 | 10,8 | 12,6 | 58,5 | 18,1 |
| 950 | 85,5 | 11,7 | 13,5 | 60,3 | 14,5 |
| 1000 | 89,1 | 12,6 | 15,3 | 61,2 | 10,9 |
| 1050 | 87,3 | 11,7 | 14,8 | 60,8 | 12,7 |
| Bei T_{H} < 900 K nimmt die Rußbildung in der Vorheizungskammer zu. | | | | | |

**Tabelle 2**

| T_{H} = 950 K; τ_{H} = 1,5 ms; P = 3,5 MPa; τ_{B} =7 ms | | | | | |
|---|---|---|---|---|---|
| Temperatur der Gemischvorheizung, K | Methankonversionsgrad, % | Gehalt der Pyrolyseprodukte, % Mass. | | | |
| | | Wasserstoff | Azetylen | Ethylen | Sonstige Produkte |
| 1700 | 47,7 | 6,4 | 5,4 | 35,9 | 52,3 |
| 1800 | 75,1 | 10,1 | 8,5 | 56,5 | 24,9 |
| 1900 | 84,8 | 11,4 | 13,3 | 60,1 | 15,2 |
| 2100 | 89,5 | 12,2 | 20,0 | 57,3 | 16,5 |
| 2200 | 93,6 | 13,0 | 25,4 | 55,2 | 6,4 |
| Bei Tₙ < 1800 K nimmt die Rußbildung in der Reaktionskammer zu. | | | | | |

**Tabelle 3**

| T_{H} = 950 K; Tₙ= 1950 K; P = 3,5 MPa; τ_{B} =7 ms | | | | | |
|---|---|---|---|---|---|
| Gemischaufheizungszeit in der ersten Stufe der Pyrolyse, ms | Methankonversionsgrad, % | Gehalt der Pyrolyseprodukte, % Mass. | | | |
| | | Wasserstoff | Azetylen | Ethylen | Sonstige Produkte |
| 0,8 | 82,4 | 11,2 | 8,8 | 62,4 | 17,6 |
| 1,0 | 86,4 | 12,1 | 10,3 | 64,0 | 13,6 |
| 1,5 | 85,5 | 11,7 | 13,5 | 60,3 | 14,5 |
| 2,0 | 84,7 | 10,9 | 6,5 | 67,3 | 15,3 |
| 2,5 | 89,8 | 12,9 | 18,9 | 58,0 | 10,1 |

**Tabelle 4**

| T_{H} = 950 K; Tₙ= 1950 K; τ_{H} = 1,5 ms; τ_{B} =7 ms | | | | | |
|---|---|---|---|---|---|
| Druck auf der ersten Stufe der Pyrolyse, MPa | Methankonversionsgrad, % | Bestand der Pyrolyseprodukte, % Mass. | | | |
| | | Wasserstoff | Azetylen | Ethylen | Sonstige Produkte |
| 1,5 | 79,0 | 11,5 | 20,7 | 46,8 | 21,0 |
| 2,0 | 72,1 | 10,2 | 14,1 | 47,8 | 27,9 |
| 3,5 | 85, 5 | 11,7 | 13,5 | 60,3 | 14,5 |
| 4,0 | 86,1 | 11,9 | 12,8 | 61,4 | 13,9 |
| 5,0 | 88,0 | 12,0 | 12,1 | 63,9 | 12,0 |
| 6,0 | 89,8 | 12,4 | 11,7 | 65,7 | 10,2 |

**Tabelle 5**

| T_{H} = 950 K; Tₙ = 1950 K; P = 3,5 MPa; τ_{H} =1,5 ms | | | | | |
|---|---|---|---|---|---|
| Gemischhaltedauer in der zweiten Stufe der Pyrolyse, ms | Methankonversionsgrad, % | Bestand der Pyrolyseprodukte, % Mass. | | | |
| | | Wasserstoff | Azetylen | Ethylen | Sonstige Produkte |
| 4 | 86,2 | 13,9 | 18,0 | 54,3 | 13,8 |
| 5 | 84,5 | 12,5 | 16,3 | 53,7 | 15, 5 |
| 7 | 85,5 | 11,7 | 13,5 | 60,3 | 14,5 |
| 10 | 88,1 | 11,6 | 9,1 | 67,4 | 11,9 |
| 11 | 89,2 | 11,5 | 2,1 | 75,6 | 10,8 |
| Bei τ_{B} > 10 ms wird eine Reaktionskammerverkohlung beobachtet. | | | | | |

Wie aus den angeführten Ergebnisdaten ersichtlich ist, lässt die direkte Methankonversion bei den Pyrolysebedingungen gemäß der Erfindung in den Pyrolyseprodukten größtenteils Ethylen 67,4 % entstehen. Der Azetylenanteil beträgt dabei 9,1 %. Der Methankonversionsgrad ist 88,1 %, d.h., das gesetzte Ziel ist erreicht worden.

### Gewerbliche Anwendbarkeit

Die Erfindung kann in verschiedenen Industriewerken realisiert werden, die sich in der Methanverarbeitung spezialisieren. Diese Betriebe sollen verschiedene Quellen der Pyrolyseausgangsmaterialaufheizung verwenden (plasmachemisches, elektrisches, lonenstrahl-Verfahren) mit gleichzeitiger Modernisierung der vorhandenen Ausrüstung in Übereinstimmung mit der Erfindung.

## Patentansprüche

1. Verfahren zur direkten Methanpyrolyse, bei dem eine Gasvorheizung, eine Zuführung des aufgeheizten Gases zur Reaktionszone und eine Härtung der Reaktionsprodukte vorgesehen ist, **dadurch gekennzeichnet, dass** Gas auf 900-1000 K vorgeheizt und die Methankonversion in zwei Stufen durchgeführt wird, wobei in der ersten Stufe Gas auf 1900 - 2100 K in 1 - 2 ms aufgeheizt, in der zweiten Stufe das aufgeheizte Gemisch der Primärpyrolyseprodukte 5 - 10 ms unter einem Druck von 2 - 5 MPa unter adiabatischer Bedingung gehalten wird.

2. Verfahren nach Anspruch 1, **dadurch gekennzeichnet, dass** die Methanpyrolyse in der ersten Stufe durch Kompensation der Energieverluste für partielle Methankonversion bei Zuführung von Energie von außen erfolgt.

## Claims

1. A method for direct methane pyrolysis, in which gas preheating, delivery of the heated gas to the reaction zone, and hardening of the reaction products is provided, **characterized in that** gas is preheated to 900-1000 K, and the methane conversion is performed in two stages, in which in the first stage gas is heated to 1900-2100 K in 1-2 ms, and in the second stage, the heated mixture of the primary pyrolysis products is kept for 5-10 ms at a pressure of 2-5 MPa under adiabatic conditions.

2. The method as defined by claim 1, **characterized in that** the methane pyrolysis is effected in the first stage by compensation for the energy losses for partial methane conversion upon delivery of energy from outside.

## Revendications

1. Procédé pour la pyrolyse directe du méthane, dans lequel on prévoit un préchauffage du gaz, un apport du gaz chauffé vers la zone de réaction et un durcissement des produits réactionnels, **caractérisé en ce que** le gaz est préchauffé à 900-1000 K et la conversion méthanique est effectuée en deux étapes, du gaz étant chauffé à 1900-2100 K en 1-2 ms dans la première étape et le mélange chauffé des produits primaires de pyrolyse étant maintenu sous une pression de 2-5 MPa pendant 5-10 ms sous condition adiabatique.

2. Procédé selon la revendication 1, **caractérisé en ce que** la pyrolyse du méthane est réalisée dans la première étape par compensation des pertes énergétiques pour la conversion partielle du méthane par apport externe d'énergie.
